# FASCICULE DE BREVET EUROPEEN

(11) **EP 1 928 448 B1**
(45) Date de publication et mention de la délivrance du brevet: **02.09.2009**
(21) Numéro de dépôt: 06808148.8
(22) Date de dépôt: 18.09.2006
(51) Int. Cl.: A61K 31/192, A61K 31/196, A61K 31/222, A61K 31/138, A61K 31/343, A61K 31/135, A61K 31/136, A61K 31/4353, A61P 25/22, A61P 25/24

(54) **ASSOCIATION D'AGONISTE AUX RECEPTEURS ß3 ET D'INHIBITEURS DE LA RECAPTURE DE MONOAMINES, COMPOSITION PHARMACEUTIQUE LA CONTENANT ET SON UTILISATION EN THERAPEUTIQUE**
VERBINDUNG EINES ß3-REZEPTOR-AGONISTEN MIT MONOAMIN-WIEDERAUFNAHMEHEMMERN, PHARMAZEUTISCHE ZUSAMMENSETZUNG DAMIT UND THERAPEUTISCHE VERWENDUNG DAFÜR
ASSOCIATION OF ß3 RECEPTOR AGONIST AND MONOAMINE REUPTAKE INHIBITORS, PHARMACEUTICAL COMPOSITION AND THERAPEUTIC USE THEREOF

(30) Priorité: 19.09.2005 FR 0509528
(43) Date de publication de la demande: 11.06.2008
(73) Titulaire: Sanofi-Aventis, 75013 Paris (FR)
(72) Inventeur: GRIEBEL, Guy, F-92260 Fontenay aux Roses (FR); STEMMELIN, Jeanne, F-75009 Paris (FR)
(74) Mandataire: Morel-Pécheux, Muriel
(86) Numéro de dépôt international: PCT/FR2006/002125
(87) Numéro de publication internationale: WO 2007/034056

(56) Documents cités:
- WO-A-20/05025563
- US-A- 5 270 341
- US-A- 5 288 749
- US-A- 6 159 971
- US-A1- 2004 180 953
- LENARD NATALIE R ET AL: "Activation of beta2- and beta3-adrenergic receptors increases brain tryptophan." THE JOURNAL OF PHARMACOLOGY AND EXPERIMENTAL THERAPEUTICS. MAY 2003, vol. 305, no. 2, mai 2003 (2003-05), pages 653-659, XP002382698 ISSN: 0022-3565

## Description

L'invention se rapporte à l'association d'au moins un agoniste des récepteurs β3 adrénergiques (ou agoniste β3) avec un inhibiteur de la recapture de monoamines (IRMA).

L'invention se rapporte également à une composition pharmaceutique comprenant l'association de l'invention et son utilisation en thérapeutique.

Les phényléthanolaminotétralines sont connues en tant qu'agents lypolytiques et spasmolytiques intestinaux agissant par un mécanisme de simulation sélective de récepteurs β atypiques (ni β1, ni β2) présents dans l'intestin. Leur préparation et leur caractérisation ont été décrites en particulier dans les documents EP-A-211721, EP-A-303545, EP-A-303546 et EP-A-383686.

L'utilisation comme antidépresseurs d'agonistes des récepteurs adrénergiques β3 tels que les phényléthanolaminotétralines a également été décrite dans le document EP-A-489640.

Parmi les inhibiteurs de la recapture de monoamines, les inhibiteurs de la recapture de sérotonine et noradrénaline sont connus en tant qu'agents antidépresseurs et sont décrits notamment dans le document EP-A-958824.

Il existe toujours un besoin en des médicaments encore plus performants pour traiter les patients souffrants de dépression ou d'anxiété.

L'invention vise à répondre à ce but, en proposant une association d'un agoniste aux récepteurs β3 et d'un inhibiteur de la recapture de monoamines, cette association présentant une action améliorée par rapport à l'action des deux principes actifs pris individuellement.

Un premier objet de l'invention concerne donc une telle association.

Un deuxième objet de l'invention concerne une composition pharmaceutique comprenant, à titre de principe actif, une telle association.

Un troisième objet de l'invention se rapporte à l'utilisation d'une telle association pour le traitement et/ou la prévention de la dépression ou de l'anxiété.

Ainsi, un premier aspect de l'invention se rapporte à l'association d'au moins un agoniste des récepteurs β3 adrénergiques avec au moins un IRMA.

Au sens de la présente invention, on entend par le terme général IRMA les inhibiteurs de la recapture de monamines. A titre d'exemple, on peut citer notamment les inhibiteurs sélectifs de la recapture de sérotonine (SSRI), les inhibiteurs sélectifs de la recapture de noradrénaline (SNRI), les inhibiteurs de la recapture de dopamine, les inhibiteurs de la recapture de dopamine et de noradrénaline, ainsi que les inhibiteurs de la recapture de sérotonine et de noradrénaline.

Selon un premier mode d'exécution de l'invention, l'agoniste aux récepteurs β3 adrénergiques est une phényléthanolaminotétraline de formule générale (I) : 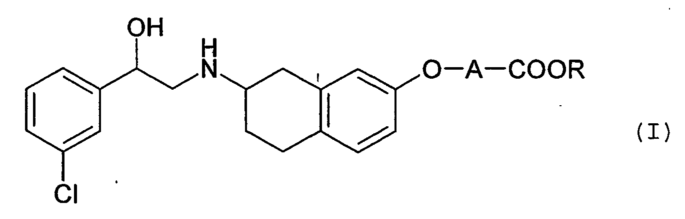 dans laquelle :
A représente un groupe C₁₋₄ alkylène, et
R représente un atome d'hydrogène ou un groupe C₁₋₄ alkyle, sous forme de base ou de sel d'addition à un acide.

Les sels utilisables peuvent être préparés avec des acides pharmaceutiquement acceptables, mais les sels d'autres acides utiles, par exemple, pour la purification ou l'isolement des phényléthanolaminotétralines de formule (I), font également partie de l'invention.

Les phényléthanolaminotétralines de formule générale (I) peuvent exister sous forme d'hydrates ou de solvates, à savoir sous forme d'associations ou de combinaisons avec une ou plusieurs molécules d'eau ou avec un solvant. De tels hydrates et solvates font également partie de l'invention.

Les phényléthanolaminotétralines de formule générale (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Dans le cadre de la présente invention, on entend par
- C_{t-z} où t et z peuvent prendre les valeurs de 1 à 4, une chaîne carbonée pouvant avoir de t à z atomes de carbone, par exemple C₁₋₄ une chaîne carbonée pouvant avoir de 1 à 4 atomes de carbone ;
- un groupe alkylène : un groupe alkyle divalent saturé, linéaire, ramifié ou cyclique, par exemple un groupe C₁₋₄-alkylène représente une chaîne carbonée divalente de 1 à 4 atomes de carbone, linéaire ou ramifiée, par exemple un méthylène, éthylène, 1-méthyléthylène, propylène, isopropylidène, butylène ;
- un groupe alkyle : un groupe aliphatique saturé, linéaire, ramifié ou cyclique ; par exemple un groupe C₁₋₄-alkyle représente une chaîne carbonée qui peut avoir de 1 à 4 atomes de carbone, par exemple un méthyle, éthyle, propyle, isopropyle, butyle, isobutyle, tert-butyle.

Un premier groupe de phényléthanolaminotétralines utilisables selon l'invention est celui de formule (I) dans laquelle :
A représente un groupement méthylène ou isopropylidène et R représente un atome d'hydrogène ou un groupe C₁₋₄ alkyle.

Parmi les phényléthanolaminotétralines utilisables dans le cadre de l'invention, on peut citer notamment le [2-(3-chlorophenyl)-2-hydroxyéthylamino-5,6,7,8-tetrahydronaphtalen-2-yloxy]acétate d'éthyle.

Parmi les diastéréoisomères du [2-(3-chlorophenyl)-2-hydroxyéthylamino-5,6,7,8-tetrahydronaphtalen-2-yloxy]acétate d'éthyle, on peut notamment citer le [(7S)-7(2R)-2-(3-chlorophenyl)-2-hydroxyéthylamino-5,6,7,8-tetrahydronaphtalen-2-yloxy]acétate d'éthyle.

Des exemples de sel de phényléthanolaminotétraline utilisables selon l'invention sont le chlorhydrate de [2-(3-chlorophenyl)-2-hydroxyéthylamino-5,6,7,8-tetrahydronaphtalen-2-yloxy]acétate d'éthyle et le chlorhydrate de [(7S)-7(2R)-2-(3-chlorophenyl)-2-hydroxyéthylamino-5,6,7,8-tetrahydronaphtalen-2-yloxy]acétate d'éthyle.

Les composés ci-dessus sont décrits notamment dans le document EP-A-303546.

Le chlorhydrate de [(7S)-7(2R)-2-(3-chlorophenyl)-2-hydroxyéthylamino-5,6,7,8-tetrahydronaphtalen-2-yloxy]acétate d'éthyle peut également se présenter sous une forme cristalline particulière, appelée forme B (également appelée forme 2). La forme B, qui est décrite dans le document EP-A-1404641 présente notamment les caractéristiques suivantes :
- pics d'absorption IR caractéristiques (cm⁻¹) : 2780, 2736, 1722, 1211 ;
- point de fusion : 129 ± 2°C ;
- raies caractéristiques du diagramme de diffraction des rayons X sur poudre (à 0,1(2θ) près) : 7,69 - 9,83 - 13,95 - 16,58 - 18,70 - 20,40 - 21,57 - 23,40 - 24,15 - 25,64.

La forme B du chlorhydrate de [(7S)-7(2R)-2-(3-chlorophenyl)-2-hydroxyéthylamino-5,6,7,8-tetrahydronaphtalen-2-yloxy]acétate d'éthyle correspond généralement à un produit comprenant au moins 95% en poids, plus particulièrement 99% en poids de la forme B à côté des autres formes polymorphes.

La forme B du chlorhydrate de [(7S)-7(2R)-2-(3-chlorophenyl)-2-hydroxyéthylamino-5,6,7,8-tetrahydronaphtalen-2-yloxy]acétate d'éthyle est un autre exemple de phényléthanolaminotétraline utilisable dans le cadre de l'invention.

Selon un autre mode d'exécution de l'invention, l'agoniste aux récepteurs β3 adrénergiques est choisi parmi les aryléthanoldiamines sous forme de base ou de sel d'addition à un acide, telles que celles décrites dans le document WO 02066418, par exemple l'acide 3'-[[2-[[(2R)-2-(3-chlorophényl)-2-hydroxyéthyl]amino]éthyl]amino]biphényl-3-carboxylique ou son sel de chlorhydrate.

L'association selon l'invention comprend également un IRMA, sous forme de base ou de sel d'addition à un acide.

L'IRMA peut être choisi notamment parmi la fluoxétine, le chlorhydrate de fluoxétine, le citalopram, le bromhydrate de citaprolam, la fluvoxamine, le maléate de fluvoxamine, la paroxétine, le chlorhydrate de paroxétine, la sertraline, le chlorhydrate de sertraline, le milnacipran, le chlorhydrate de milnacipran, le escitalopram (S-citalopram), l'oxalate de escitalopram, la duloxétine, le chlorhydrate de duloxétine, la venlafaxine, le chlorhydrate de venlafaxine, la desvenlafaxine, la radafaxine, le bupropion, le chlorhydrate de bupropion, ainsi que leurs mélanges.
- La fluoxétine ou N-méthyle-3-(p-trifluoromethoxyphenoxy)-3-phenylpropalamine, est commercialisée sous forme de chlorhydrate et en tant que mélange racémique des deux énantiomères R et S.
   Au sens de la présente invention, on entend par fluoxétine, le composé sous forme de base libre ou de sel d'addition acide, incluant également le mélange de racémates ou les énantiomères R ou S seuls.
- La duloxétine ou N-méthyl-3-(1-naphtalenyloxy)-3-(2-thiènyl)propanamine, est également connue et est généralement administrée sous forme de chlorhydrate de l'énantiomère (+).
- La venlafaxine est décrite dans la littérature. Son procédé de synthèse ainsi que son activité comme inhibiteur de recapture de sérotonine et de norépinéphrine sont décrits dans le document U.S. 4,761,501.
- Le milnacipran ou N,N-diéthyl-2-aminométhyl-1-phenylcyclopropanecarboxamide, est décrit dans le document U.S 4,478,836. L'activité pharmacologique du milnacipran comme inhibiteur de la recapture de sérotonine et de la noradrénaline est décrite par Moret et al, Neuropharmacology 24, 1211-19 (1985).
- Le citalopram ou 1-[3-(diméthylamino)propyl]-1-(4-fluorophényl)-1,3-dihydro-5-isobenzofurancarbonitrile, est décrit dans le document U.S. 4,136,193.
- Le escitalopram (S-citalopram) ou (+)-1-[3-(Dimethylamino)propyl]-1-(4-fluorophenyl)-1,3-dihydro-5-isobenzofurancarbonitrile est décrit dans le document EP 0347066.
- La fluvoxamine ou 5-méthoxy-1-[4-(trifluorométhyl)-phényl]-1-pentanone O-(2-aminoéthyl)oxime, est décrite dans le document U.S. 4,085,225.
- La paroxétine ou trans-(-)-3-[(1,3-benzodioxol-5-yloxy)méthyl]-4-(4-fluorophényl)pipéridine est décrite dans les documents U.S. 3,912,743 et U.S. 4,007,196.
- La sertraline, hydrochlorate de (1S-cis)-4-(3,4-dichlorophényl)-1,2,3,4-tétrahydro-N-méthyl-1-naphtylamine, est un inhibiteur de la recapture de sérotonine, commercialisé comme antidépresseur. Ce composé est décrit dans le document U.S. 4,536,518.
- la desvenlafaxine ou 4[(1RS)-2-(diméthylamino)-1-(1-hyroxycyclohexyl)éthyl]phénol ainsi que la radafaxine sont décrites dans la littérature.
- le bupropion ou 1-(3chlorophényl)-2-[(1,1-diméthyléthyl)amino]-1-propanone est décrit dans le document US 3,819,706.

Selon une variante d'exécution, des IRMA utilisables selon l'invention sont les inhibiteurs de la recapture de sérotonine, par exemple la fluoxétine et le escitaprolam.

Ainsi, un premier exemple d'association selon l'invention est l'association constituée de chlorhydrate de [(7S)-7(2R)-2-(3-chlorophenyl)-2-hydroxyéthylamino-5,6,7,8-tetrahydronaphtalen-2-yloxy]acétate d'éthyle et de fluoxétine, par exemple de chlorhydrate de fluoxétine.

Un autre exemple d'association selon l'invention est l'association constituée de chlorhydrate de [(7S)-7(2R)-2-(3-chlorophenyl)-2-hydroxyéthylamino-5,6,7,8-tetrahydronaphtalen-2-yloxy]acétate d'éthyle et de escitalopram, par exemple d'oxalate de escitalopram.

Un second objet de l'invention concerne une composition pharmaceutique comprenant, à titre de principe actif, une association telle que définie précédemment, et un ou plusieurs excipients pharmaceutiquement acceptables.

La composition pharmaceutique contient une dose efficace de chaque principe actif présent dans l'association selon l'invention.

Les excipients sont choisis selon la forme pharmaceutique et le mode d'administration souhaités, parmi les excipients habituels qui sont connus de l'homme du métier.

La composition peut être administrée par voie orale, parentérale ou rectale.
Les formes unitaires d'administration appropriées comprennent les formes par voie orale telles que les comprimés, les gélules molles ou dures, les poudres, les granules et les solutions ou suspensions orales, les formes d'administration sublinguale, buccale, intrachéale, intraoculaire, intranasale, par inhalation, les formes d'administration topique, transdermique, sous-cutanée, intra-musculaire ou intraveineuse, les formes d'administration rectale et les implants. Pour l'application topique, on peut utiliser les principes actifs selon l'invention dans des crèmes, gels, pommades ou lotions.

Selon l'invention, les deux principes actifs sont administrés selon la même voie, par exemple la voie orale, ou l'un des principes actifs est administré selon une première voie, par exemple la voie orale, et l'autre principe actif est administré selon une voie différente, par exemple la voie parentérale.

Lorsqu'on prépare une composition sous forme de comprimé, on mélange les ingrédients actifs avec un ou plusieurs excipients pharmaceutiques, tels que la gélatine, l'amidon, le lactose, le stéarate de magnésium, le talc, la silice, la gomme arabique, le mannitol, la cellulose microcristalline, l'hypromellose ou analogues.
On peut enrober les comprimés de saccharose, d'un dérivé cellulosique ou d'autres matières adaptées à l'enrobage. Les comprimés peuvent être réalisés par différentes techniques, telles que la compression directe, la granulation sèche ou humide ou la fusion à chaud.
On peut également obtenir une composition pharmaceutique sous forme de gélule en mélangeant les ingrédients actifs avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents pharmacologiquement compatibles, par exemple le propylèneglycol ou le butylèneglycol.

Selon la pratique habituelle, le dosage approprié à chaque patient est déterminé par le médecin selon le mode d'administration, l'âge, le poids et la réponse dudit patient.

Les doses dépendent de l'effet recherché, de la durée du traitement et de la voie d'administration utilisée.

Par exemple par voie orale, les doses journalières de chacun des principes actifs de l'association selon l'invention sont les suivantes :
- agoniste aux récepteurs β3 : entre 10 et 2000 mg par jour et par personne, notamment entre 50 et 1000 mg par jour et par personne ;
- IRMA : entre 5 et 800 mg par jour et par personne, notamment entre 10 et 500 mg par jour et par personne.

Il peut y avoir des cas particuliers où des dosages plus élevés ou plus faibles sont appropriés. De tels dosages ne sortent pas du cadre de l'invention.

Les doses respectives de l'agoniste β3 et d'IRMA sont généralement sensiblement identiques entre elles ou bien peuvent différer l'une de l'autre.

A titre d'exemple, une forme unitaire d'administration de l'agoniste aux récepteurs β3 sous forme de comprimé comprend les ingrédients suivants :

| | |
|---|---|
| Chlorhydrate de [(7S)-7(2R)-2-(3-chlorophenyl)-2-hydroxyéthylamino-5,6,7,8-tetrahydronaphtalen-2-yloxy] acétate d'éthyle | 50 mg |
| Mannitol | 174 mg |
| Croscarmellose sodique | 6 mg |
| Amidon de maïs | 15 mg |
| Hydroxypropyl-méthylcellulose | 2 mg |
| Stéarate de magnésium | 3 mg |

Egalement à titre d'exemple, une forme unitaire d'administration d'escitalopram sous forme de comprimé peut comprendre 10 mg d'oxalate d'escitalopram et des excipients usuels, par exemple de la cellulose microcristalline, de la silice colloïdale, du talc, de la croscarmellose sodique, du stéarate de magnésium.

L'administration de chacun des principes actifs peut également être effectuée de façon simultanée, séparée ou de manière étalée dans le temps (administration séquentielle).

Lorsque l'administration est effectuée de façon simultanée, les deux principes actifs peuvent être réunis au sein d'une composition pharmaceutique unique, comprenant les deux principes actifs, telle qu'un comprimé ou une gélule.

Les deux principes actifs peuvent également, que leur administration soit simultanée ou non, être présents dans des compositions pharmaceutiques distinctes. A cet effet, l'association selon l'invention peut se présenter sous forme de kit comprenant, d'une part, au moins un agoniste β3 tel que défini dans ce qui précède et, d'autre part, au moins un IRMA tel que défini dans ce qui précède, l'agoniste aux récepteurs β3 et l'IRMA étant dans des compartiments distincts et étant destinés à être administrés de façon simultanée, séparée ou étalée dans le temps (administration séquentielle).

Un autre objet de l'invention concerne l'utilisation d'une association telle que décrite précédemment pour la préparation d'un médicament destiné au traitement et/ou à la prévention de la dépression ou de l'anxiété.

Les effets antidépresseurs de l'association salon l'invention ont été évalués chez la souris à partir du protocole de stress chronique modéré (SCM). Ce test a été développé chez le rat par Willner (P. Willner, Neuroscience and Biobehavioral Review, 1992, 16, 525-34) et adapté à la souris par Kopp et al.(C. Kopp, Behavioural Pharmacology, 1999, 10, 73-83).
Des similitudes entre l'état dépressif chez l'homme et l'effet du stress chronique modéré chez la souris ont été établies. L'émotivité des souris stressées par le SCM a été évaluée dans le test de nage forcée.

Le SCM est conduit de la manière suivante :
Des souris mâles BALB/c ByJIco (Iffa Credo Lyon, France) âgées de 8 semaines sont placées dans des cages d'expérimentation individuelles (26 X 20 X 14 cm) pourvues en nourriture et en eau à volonté, à la température contrôlée de 22±1°C.
Dans le cadre du protocole, les souris sont soumises à une série de stress tels que des bains forcés, une privation de nourriture et/ou d'eau, un hébergement dans des cages contenant de la sciure humide, une permutation du cycle jour/nuit, un maintien en illumination constante ou en obscurité, chacune de ces restrictions pouvant aller de 2 heures à 24 heures.

Un premier groupe d'animaux contrôle n'est pas soumis au SCM (groupe 1 - non stressé).
Les animaux soumis au SCM sont divisés en 4 groupes au jour 15 :
- Le groupe 2 (stress saline) est constitué d'animaux contrôles recevant une injection intrapéritonéale par jour de liquide physiologique.
- Le groupe 3 (stress fluoxétine) reçoit 1 injection intrapéritonéale par jour de chlorhydrate de fluoxétine à raison de 3mg/kg.
- Le groupe 4 (stress β3 agoniste) reçoit 1 injection intrapéritonéale par jour de chlorhydrate de [(7S)-7(2R)-2-(3-chlorophenyl)-2-hydroxyéthylamino-5,6,7,8-tetrahydronaphtalen-2-yloxy]acétate d'éthyle à raison de 1mg/kg.
- Le groupe 5 (stress fluoxétine + β3 agoniste) reçoit 1 injection intra péritonéale par jour d'une solution contenant du chlorhydrate de fluoxétine (3mg/kg) et du chlorhydrate de [(7S)-7(2R)-2-(3-chlorophenyl)-2-hydroxyéthylamino-5,6,7,8-tetrahydronaphtalen-2-yloxy]acétate d'éthyle (1mg/kg).
Les injections sont réalisées du jour 15 au jour 50.

### Emotivité lors du test de nage forcée.

Au jour 50, chaque groupe est soumis au test de nage forcée. Ce test consiste à baigner chaque animal dans un cylindre en verre contenant de l'eau et à évaluer le degré d'adaptation au stress dont fait preuve la souris. Le temps d'immobilité est mesuré sur une période de 5 minutes.
Les résultats sont reportés dans le tableau 1 ci-après.

**Tableau 1**

| Groupe | Temps d'immobilité (secondes) |
|---|---|
| 1 (non stressé) | 146,8 |
| 2 (stress saline) | 193,9 |
| 3 (stressé fluoxétine) | 178,4 |
| 4 (stressé β3 agoniste) | 182,0 |
| 5 (stressé fluoxétine + β3 agoniste) | 153,6 |

Le pourcentage de temps d'immobilité des groupes d'animaux stressés par rapport aux animaux non stressés est également calculé et indiqué dans le tableau 2 ci-après.

**Tableau 2**

| Groupe | Pourcentage de temps |
|---|---|
| **1 (non stressé)** | - |
| **2 (stressé saline)** | + 32,1 % |
| **3 (stressé fluoxétine)** | + 21,5 % |
| **4 (stressé β3 agoniste)** | + 25,1 % |
| **5 (stressé fluoxétine + β3 agoniste)** | + 4 % |

Ces résultats montrent une augmentation du temps d'immobilité des animaux stressés par rapport aux animaux non stressés suite à une exposition au SCM. L'administration répétée de β3 agoniste seul ou de fluoxétine seule ne modifie pas de manière significative ce paramètre.
Par ailleurs, l'administration répétée de l'association fluoxétine et [(7S)-7(2R)-2-(3-chlorophenyl)-2-hydroxyéthylamino-5,6,7,8-tetrahydronaphtalen-2-yloxy]acétate d'éthyle produit une réduction marquée du temps d'immobilité comparativement aux autres groupes d'animaux stressés (groupes 2 à 4).
Le tableau 2 montre également que les performances des souris traitées de manière répétée par l'association fluoxétine et [(7S)-7(2R)-2-(3-chlorophenyl)-2-hydroxyéthylamino-5,6,7,8-tetrahydronaphtalen-2-yloxy]acétate d'éthyle sont du même niveau que les performances des souris non stressées.

Les expérimentations ci-dessus montrent donc que l'invention produit un effet de type antidépresseur supérieur en terme d'efficacité à chacun des principes actifs administrés de façon isolée et au même dosage.

## Revendications

1. Association d'au moins un agoniste aux récepteurs β3 adrénergiques choisi parmi les aryléthanoldiamines sous forme de bases ou de sels d'addition à un acide, notamment l'acide 3'-[[2-[[(2R)-2-(3-chlorophényl)-2-hydroxyéthyl]amino]éthyl]amino]biphényl-3-carboxylique ou son sel de chlorhydrate, et les phényléthanolaminotétralines de formule générale (I) 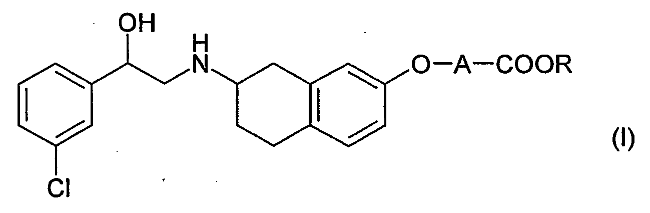 dans laquelle :
A représente un groupe C₁₋₄ alkylène, et
R représente un atome d'hydrogène ou un groupe C₁₋₄ alkyle, sous forme de base ou de sel d'addition à un acide, ainsi qu'à l'état d'hydrate ou de solvate, avec au moins un inhibiteur de la recapture de monoamines (IRMA).

2. Association selon la revendication 1, **caractérisée en ce que** la phényléthanolaminotétraline répond à la formule générale (I) 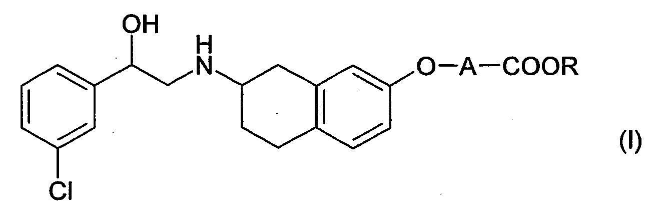 dans laquelle :
A représente un groupement méthylène ou isopropylidène, et
R représente un atome d'hydrogène ou un groupe C₁₋₄ alkyle.

3. Association selon la revendication 1 ou 2, **caractérisée en ce que** la phényléthanolaminotétraline est le [2-(3-chlorophenyl)-2-hydroxyéthylamino-5,6,7,8-tetrahydronaphtalen-2-yloxy]acétate d'éthyle.

4. Association selon l'une quelconque des revendications 1 à 3, **caractérisée en ce que** la phényléthanolaminotétraline est le chlorhydrate de [2-(3-chlorophenyl)-2-hydroxyéthylamino-5,6,7,8-tetrahydronaphtalen-2-yloxy]acétate d'éthyle.

5. Association selon l'une quelconque des revendications 1 à 4, **caractérisée en ce que** la phényléthanolaminotétraline est le [(7S)-7(2R)-2-(3-chlorophenyl)-2-hydroxyéthylamino-5,6,7,8-tetrahydronaphtalen-2-yloxy]acétate d'éthyle.

6. Association selon l'une quelconque des revendications 1 à 5, **caractérisée en ce que** la phényléthanolaminotétraline est le chlorhydrate de [(7S)-7(2R)-2-(3-chlorophenyl)-2-hydroxyéthylamino-5,6,7,8-tetrahydronaphtalen-2-yloxy]acétate d'éthyle.

7. Association selon l'une quelconque des revendications 1 à 6, **caractérisée en ce que** la phényléthanolaminotétraline est le chlorhydrate de [(7S)-7(2R)-2-(3-chlorophenyl)-2-hydroxyéthylamino-5,6,7,8-tetrahydronaphtalen-2-yloxy]acétate d'éthyle sous forme B, dont le spectre infrarouge présente les pics d'absorption caractéristiques suivants : 2780, 2736, 1722, 1211 cm⁻¹.

8. Association selon l'une quelconque des revendications 1 à 7, **caractérisée en ce que** l'IRMA est choisi parmi notamment la fluoxétine, le chlorhydrate de fluoxétine, le citalopram, le bromhydrate de citaprolam, la fluvoxamine, le maléate de fluvoxamine, la paroxétine, le chlorhydrate de paroxétine, la sertraline, le chlorhydrate de sertraline, le milnacipran, le chlorhydrate de milnacipran, le escitalopram (S-citalopram), l'oxalate de escitalopram, la duloxétine, le chlorhydrate de duloxétine, la venlafaxine, le chlorhydrate de venlafaxine, la desvenlafaxine, la radafaxine, le bupropion, le chlorhydrate de bupropion, ainsi que leurs mélanges.

9. Association selon la revendication 8, **caractérisée en ce que** l'IRMA est choisi parmi la fluoxétine, par exemple le chlorhydrate de fluoxétine, le escitalopram, par exemple l'oxalate de escitalopram.

10. Association selon l'une quelconque des revendications 1 à 9, **caractérisée en ce que** l'agoniste β3 est le chlorhydrate de [(7S)-7(2R)-2-(3-chlorophenyl)-2-hydroxyéthylamino-5,6,7,8-tetrahydronaphtalen-2-yloxy]acétate d'éthyle et l'IRMA est la fluoxétine, par exemple le chlorhydrate de fluoxétine.

11. Association selon l'une quelconque des revendications 1 à 9, **caractérisée en ce que** l'agoniste β3 est le chlorhydrate de [(7S)-7(2R)-2-(3-chlorophenyl)-2-hydroxyéthylamino-5,6,7,8-tetrahydronaphtalen-2-yloxy]acétate d'éthyle et l'IRMA est le escitalopram, par exemple l'oxalate de escitalopram.

12. Composition pharmaceutique comprenant à titre de principes actifs, au moins un agoniste aux récepteurs β3 adrénergiques choisi parmi les phényléthanolaminotétralines de formule générale (I) 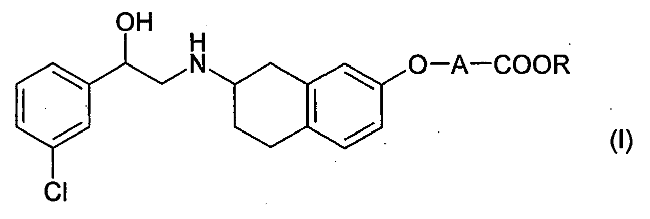 dans laquelle :
A représente un groupe C₁₋₄ alkylène, et
R représente un atome d'hydrogène ou un groupe C₁₋₄ alkyle, sous forme de base ou de sel d'addition à un acide, ainsi qu'à l'état d'hydrate ou de solvate et au moins un IRMA, ainsi qu'au moins un excipient pharmaceutiquement acceptable.

13. Composition pharmaceutique selon la revendication 12, **caractérisée en ce que** la phényléthanolaminotétraline répond à la formule générale (I) dans laquelle :
A représente un groupe méthylène ou isopropylidène, et
R représente un atome d'hydrogène ou un groupe C₁₋₄ alkyle.

14. Composition pharmaceutique selon l'une quelconque des revendications 12 ou 13, **caractérisée en ce que** la phényléthanolaminotétraline est le [2-(3-chlorophenyl)-2-hydroxyéthylamino-5,6,7,8-tetrahydronaphtalen-2-yloxy]acétate d'éthyle, le [(7S)-7(2R)-2-(3-chlorophenyl)-2-hydroxyéthylamino-5,6,7,8-tetrahydronaphtalen-2-yloxy]acétate d'éthyle, le chlorhydrate de [2-(3-chlorophenyl)-2-hydroxyéthylamino-5,6,7,8-tetrahydronaphtalen-2-yloxy]acétate d'éthyle, le chlorhydrate de [(7S)-7(2R)-2-(3-chlorophenyl)-2-hydroxyéthylamino-5,6,7,8-tetrahydronaphtalen-2-yloxy]acétate d'éthyle ou le chlorhydrate de [(7S)-7(2R)-2-(3-chlorophenyl)-2-hydroxyéthylamino-5,6,7,8-tetrahydronaphtalen-2-yloxy]acétate d'éthyle sous forme B.

15. Composition pharmaceutique selon l'une quelconque des revendications 12 à 14, **caractérisée en ce que** l'IRMA est choisi parmi notamment fluoxétine, le chlorhydrate de fluoxétine, le citalopram, le bromhydrate de citaprolam, la fluvoxamine, le maléate de fluvoxamine, la paroxétine, le chlorhydrate de paroxétine, la sertraline, le chlorhydrate de sertraline, le milnacipran, le chlorhydrate de milnacipran, le escitalopram (S-citalopram), l'oxalate de escitalopram, la duloxétine, le chlorhydrate de duloxétine, la venlafaxine, le chlorhydrate de venlafaxine, la desvenlafaxine, la radafaxine, le bupropion, le chlorhydrate de bupropion, ainsi que leurs mélanges.

16. Composition pharmaceutique selon la revendication 15, **caractérisée en ce que** l'IRMA est la fluoxétine ou le escitalopram.

17. Composition pharmaceutique selon l'une quelconque des revendications 12 à 16, pour une utilisation simultanée, séparée ou étalée dans le temps.

18. Kit comprenant, d'une part, au moins un agoniste aux récepteurs β3 adrénergiques tel que défini dans l'une quelconque des revendications 1 à 7, et d'autre part, au moins un IRMA tel que défini selon la revendication 8, l'agoniste aux récepteurs β3 adrénergiques et l'IRMA étant dans des compartiments distincts et étant destinés à être administrés de façon simultanée, séparée ou étalée dans le temps (administration séquentielle).

19. Utilisation d'une association d'au moins un agoniste aux récepteurs β3 adrénergiques choisi parmi les aryléthanoldiamines sous forme de bases ou de sels d'addition à un acide, notamment l'acide 3'-[[2-[[(2R)-2-(3-chlorophényl)-2-hydroxyéthyl]amino]éthyl]amino]biphényl-3-carboxylique ou son sel de chlorhydrate, et les phényléthanolaminotétralines de formule générale (I) 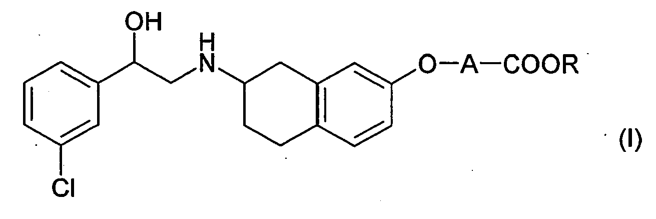 dans laquelle :
A représente un groupe C₁₋₄ alkylène, et
R représente un atome d'hydrogène ou un groupe C₁₋₄ alkyle, sous forme de base ou de sel d'addition à un acide, ainsi qu'à l'état d'hydrate ou de solvate avec au moins un IRMA, pour la préparation d'un médicament destiné au traitement et/ou à la prévention de la dépression ou de l'anxiété.

20. Utilisation selon la revendication 19, **caractérisée en ce que** l'association est telle que définie selon l'une quelconque des revendications 2 à 11.

## Claims

1. Combination of at least one β3 adrenergic receptor agonist chosen from arylethanoldiamines in the form of bases or of addition salts with an acid, in particular 3'-[[2-[[(2R)-2-(3-chlorophenyl)-2-hydroxyethyl]amino]-ethyl]amino]biphenyl-3-carboxylic acid or the hydrochloride salt thereof, and the phenylethanolaminotetralins of general formula (I) 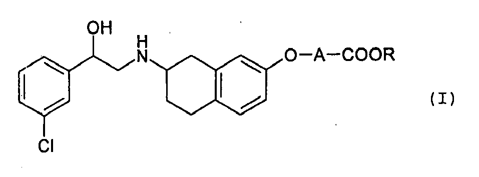 in which:
A represents a C₁-C₄ alkylene group, and
R represents a hydrogen atom or a C₁-C₄ alkyl group, in the form of a base or of an addition salt with an acid, and also in the form of a hydrate or of a solvate, with at least one monoamine reuptake inhibitor (MARI).

2. Combination according to Claim 1, **characterized in that** the phenylethanolaminotetralin corresponds to general formula (I) 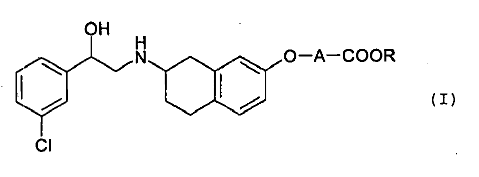 in which:
A represents a methylene or isopropylidene group, and
R represents a hydrogen atom or a C₁-C₄ alkyl group.

3. Combination according to Claim 1 or 2, **characterized in that** the phenylethanolaminotetralin is ethyl [2-(3-chlorophenyl)-2-hydroxyethylamino-5,6,7,8-tetrahydronaphthalen-2-yloxy]acetate.

4. Combination according to any one of Claims 1 to 3, **characterized in that** the phenylethanolaminotetralin is ethyl [2-(3-chlorophenyl)-2-hydroxyethylamino-5,6,7,8-tetrahydronaphthalen-2-yloxy]acetate hydrochloride.

5. Combination according to any one of Claims 1 to 4, **characterized in that** the phenylethanolaminotetralin is ethyl [(7S)-7(2R)-2-(3-chlorophenyl)-2-hydroxyethylamino-5,6,7,8-tetrahydronaphthalen-2-yloxy]acetate.

6. Combination according to any one of Claims 1 to 5, **characterized in that** the phenylethanolaminotetralin is ethyl [(7S)-7(2R)-2-(3-chlorophenyl)-2-hydroxyethylamino-5,6,7,8-tetrahydronaphthalen-2-yloxy]acetate hydrochloride.

7. Combination according to any one of Claims 1 to 6, **characterized in that** the phenylethanolaminotetralin is ethyl [(7S)-7(2R)-2-(3-chlorophenyl)-2-hydroxyethylamino-5,6,7,8-tetrahydronaphthalen-2-yloxy]acetate hydrochloride in B form, the infrared spectrum of which shows the following characteristic absorption peaks: 2780, 2736, 1722, 1211 cm⁻¹.

8. Combination according to any one of Claims 1 to 7, **characterized in that** the MARI is chosen in particular from fluoxetine, fluoxetine hydrochloride, citalopram, citalopram hydrobromide, fluvoxamine, fluvoxamine maleate, paroxetine, paroxetine hydrochloride, sertraline, sertraline hydrochloride, milnacipran, milnacipran hydrochloride, escitalopram (S-citalopram), escitalopram oxalate, duloxetine, duloxetine hydrochloride, venlafaxine, venlafaxine hydrochloride, desvenlafaxine, radafaxine, bupropion and bupropion hydrochloride, and mixtures thereof.

9. Combination according to Claim 8, **characterized in that** the MARI is chosen from fluoxetine, for example fluoxetine hydrochloride, and escitalopram, for example escitalopram oxalate.

10. Combination according to any one of Claims 1 to 9, **characterized in that** the β3 agonist is ethyl [(7S)-7(2R)-2-(3-chlorophenyl)-2-hydroxyethylamino-5,6,7,8-tetrahydronaphthalen-2-yloxy]acetate hydrochloride and the MARI is fluoxetine, for example fluoxetine hydrochloride.

11. Combination according to any one of Claims 1 to 9, **characterized in that** the β3 agonist is ethyl [(7S)-7(2R)-2-(3-chlorophenyl)-2-hydroxyethylamino-5,6,7,8-tetrahydronaphthalen-2-yloxy]acetate hydrochloride and the MARI is escitalopram, for example escitalopram oxalate.

12. Pharmaceutical composition comprising, by way of active ingredients, at least one β3 adrenergic receptor agonist chosen from the phenylethanolaminotetralins of general formula (I) 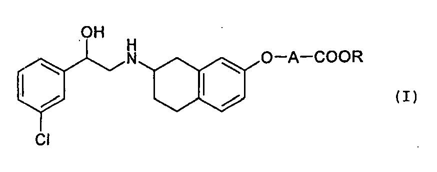 in which:
A represents a C₁-C₄ alkylene group, and
R represents a hydrogen atom or a C₁-C₄ alkyl group, in the form of a base or of an addition salt with an acid, and also in the form of a hydrate or of a solvate, and at least one MARI, and also at least one pharmaceutically acceptable excipient.

13. Pharmaceutical composition according to Claim 12, **characterized in that** the phenylethanolaminotetralin corresponds to general formula (I) in which:
A represents a methylene or isopropylidene group, and
R represents a hydrogen atom or a C₁-C₄ alkyl group.

14. Pharmaceutical composition according to either one of Claims 12 and 13, **characterized in that** the phenylethanolaminotetralin is ethyl [2-(3-chlorophenyl)-2-hydroxyethylamino-5,6,7,8-tetrahydronaphthalen-2-yloxy]acetate, ethyl [(7S)-7(2R)-2-(3-chlorophenyl)-2-hydroxyethylamino-5,6,7,8-tetrahydronaphthalen-2-yloxy]acetate, ethyl [2-(3-chlorophenyl)-2-hydroxyethylamino-5,6,7,8-tetrahydronaphthalen-2-yloxy]acetate hydrochloride, ethyl [(7S)-7(2R)-2-(3-chlorophenyl)-2-hydroxyethylamino-5,6,7,8-tetrahydronaphthalen-2-yloxy]acetate hydrochloride or ethyl [(7S)-7(2R)-2-(3-chlorophenyl)-2-hydroxyethylamino-5,6,7,8-tetrahydronaphthalen-2-yloxy]acetate hydrochloride in B form.

15. Pharmaceutical composition according to any one of Claims 12 to 14, **characterized in that** the MARI is chosen in particular from fluoxetine, fluoxetine hydrochloride, citalopram, citalopram hydrobromide, fluvoxamine, fluvoxamine maleate, paroxetine, paroxetine hydrochloride, sertraline, sertraline hydrochloride, milnacipran, milnacipran hydrochloride, escitalopram (S-citalopram), escitalopram oxalate, duloxetine, duloxetine hydrochloride, venlafaxine, venlafaxine hydrochloride, desvenlafaxine, radafaxine, bupropion and bupropion hydrochloride, and mixtures thereof.

16. Pharmaceutical composition according to Claim 15, **characterized in that** the MARI is fluoxetine or escitalopram.

17. Pharmaceutical composition according to any one of Claims 12 to 16, for use simultaneously, separately or spread out over time.

18. Kit comprising, firstly, at least one β3 adrenergic receptor agonist as defined in any one of Claims 1 to 7 and, secondly, at least one MARI as defined according to Claim 8, the β3 adrenergic receptor agonist and the MARI being in separate compartments and being intended to be administered simultaneously, separately or spread out over time (sequential administration).

19. Use of a combination of at least one β3 adrenergic receptor agonist chosen from arylethanoldiamines in the form of bases or of addition salts with an acid, in particular 3'-[[2-[[(2R)-2-(3-chlorophenyl)-2-hydroxyethyl]amino]ethyl]amino]biphenyl-3-carboxylic acid or the hydrochloride salt thereof, and the phenylethanolaminotetralins of general formula (I) 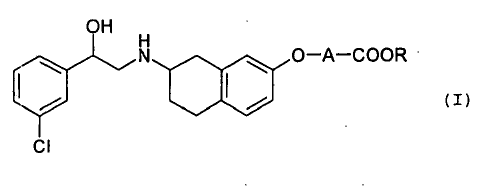 in which:
A represents a C₁-C₄ alkylene group, and
R represents a hydrogen atom or a C₁-C₄ alkyl group, in the form of a base or of an addition salt with an acid, and also in the form of a hydrate or of a solvate, with at least one MARI, in the preparation of a medicament for use in the treatment and/or prevention of depression or anxiety.

20. Use according to Claim 19, **characterized in that** the combination is as defined in any one of Claims 2 to 11.

## Patentansprüche

1. Kombination von mindestens einem Agonisten von adrenergen β3-Rezeptoren, ausgewählt unter Arylethanoldiaminen in Form von Basen oder Säureadditionssalzen, insbesondere 3'-[[2-[[(2R)-2-(3-Chlorphenyl)-2-hydroxyethyl]amino]-ethyl]amino]biphenyl-3-carbonsäure oder dessen Hydrochlorid, und Phenylethanolaminotetralinen der allgemeinen Formel (I) 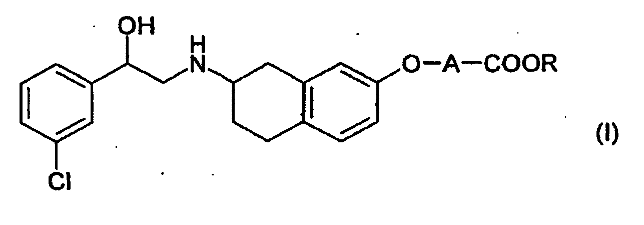 worin:
A für eine C₁₋₄-Alkylengruppe steht und
R für ein Wasserstoffatom oder eine C₁₋₄-Alkylgruppe steht, in Basenform oder Säureadditionssalzform sowie in Hydrat- oder Solvatform, mit mindestens einem Monoaminwiederaufnahmehemmer (MARI).

2. Kombination nach Anspruch 1, **dadurch gekennzeichnet, daß** das Phenylethanolaminotetralin der allgemeinen Formel (I) 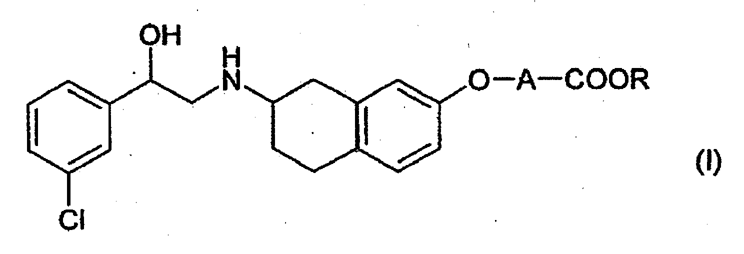 entspricht, worin:
A für eine Methylen- oder Isoproylidengruppe steht und
R für ein Wasserstoffatom oder eine C₁₋₄-Alkylgruppe steht.

3. Kombination nach Anspruch 1 oder 2, **dadurch gekennzeichnet, daß** es sich bei dem Phenylethanolaminotetralin um [2-(3-Chlorphenyl)-2-hydroxy-ethylamino-5,6,7,8-tetrahydronaphthalin-2-yloxy]-essigsäureethylester handelt.

4. Kombination nach einem der Ansprüche 1 bis 3, **dadurch gekennzeichnet, daß** es sich bei dem Phenylethanolaminotetralin um [2-(3-Chlorphenyl)-2-hydroxyethylamino-5,6,7,8-tetrahydronaphthalin-2-yloxy]essigsäureethylester-hydrochlorid handelt.

5. Kombination nach einem der Ansprüche 1 bis 4, **dadurch gekennzeichnet, daß** es sich bei dem Phenylethanolaminotetralin um [(7S)-7(2R)-2-(3-Chlorphenyl)-2-hydroxyethylamino-5,6,7,8-tetrahydronaphthalin-2-yloxy]essigsäureethylester handelt.

6. Kombination nach einem der Ansprüche 1 bis 5, **dadurch gekennzeichnet, daß** es sich bei dem Phenylethanolaminotetralin um [(7S)-7(2R)-2-(3-Chlorphenyl)-2-hydroxyethylamino-5,6,7,8-tetrahydronaphthalin-2-yloxy]essigsäureethylester-hydrochlorid handelt.

7. Kombination nach einem der Ansprüche 1 bis 6, **dadurch gekennzeichnet, daß** es sich bei dem Phenylethanolaminotetralin um [(7S)-7(2R)-2-(3-Chlorphenyl)-2-hydroxyethylamino-5,6,7,8-tetrahydronaphthalin-2-yloxy]essigsäureethylester-hydrochlorid in B-Form handelt, dessen Infrarotspektrum die folgenden charakteristischen Absorptionspeaks aufweist: 2780, 2736, 1722, 1211 cm⁻¹.

8. Kombination nach einem der Ansprüche 1 bis 7, **dadurch gekennzeichnet, daß** das MARI insbesondere unter Fluoxetin, Fluoxetin-hydrochlorid, Citalopram, Citalopram-hydrobromid, Fluvoxamin, Fluvoxamin-maleat, Paroxetin, Paroxetinhydrochlorid, Sertralin, Sertralin-hydrochlorid, Milnacipran, Milnacipran-hydrochlorid, Escitalopram (S-Citalopram), Escitalopram-oxalat, Duloxetin, Duloxetin-hydrochlorid, Venlafaxin, Venlafaxin-hydrochlorid, Desvenlafaxin, Radafaxin, Bupropion, Bupropion-hydrochlorid und Mischungen davon ausgewählt ist.

9. Kombination nach Anspruch 8, **dadurch gekennzeichnet, daß** das MARI unter Fluoxetin, beispielsweise Fluoxetin-hydrochlorid, und Escitalopram, beispielsweise Escitalopram-oxalat, ausgewählt ist.

10. Kombination nach einem der Ansprüche 1 bis 9, **dadurch gekennzeichnet, daß** es sich bei dem β3-Agonisten um [(7S)-7(2R)-2-(3-Chlorphenyl)-2-hydroxyethylamino-5,6,7,8-tetrahydronaphthalin-2-yloxy]essigsäureethylester-hydrochlorid und bei dem MARI um Fluoxetin, beispielsweise Fluoxetin-hydrochlorid, handelt.

11. Kombination nach einem der Ansprüche 1 bis 9, **dadurch gekennzeichnet, daß** es sich bei dem β3-Agonisten um [(7S)-7(2R)-2-(3-Chlorphenyl)-2-hydroxyethylamino-5,6,7,8-tetrahydronaphthalin-2-yloxy]essigsäureethylester-hydrochlorid und bei dem MARI um Escitalopram, beispielsweise Escitalopram-oxalat, handelt.

12. Pharmazeutische Zusammensetzung, die als Wirkstoffe mindestens einen Agonisten von adrenergen β3-Rezeptoren, ausgewählt unter Phenylethanolaminotetralinen der allgemeinen Formel (I) 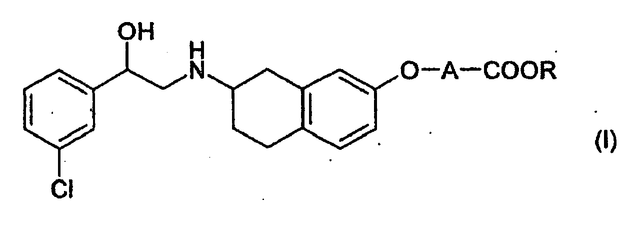 worin:
A für eine C₁₋₄-Alkylengruppe steht und
R für ein Wasserstoffatom oder eine C₁₋₄-Alkylgruppe steht, in Basenform oder Säureadditionssalzform sowie in Hydrat- oder Solvatform, und mindestens einen MARI sowie einen pharmazeutisch unbedenklichen Hilfsstoff enthält.

13. Pharmazeutische Zusammensetzung nach Anspruch 12, **dadurch gekennzeichnet, daß** das Phenylethanolaminotetralin der allgemeinen Formel (I) entspricht, worin:
A für eine Methylen- oder Isoproylidengruppe steht und
R für ein Wasserstoffatom oder eine C₁₋₄-Alkylgruppe steht.

14. Pharmazeutische Zusammensetzung nach einem der Ansprüche 12 oder 13, **dadurch gekennzeichnet, daß** es sich bei dem Phenylethanolaminotetralin um [2-(3-Chlorphenyl)-2-hydroxyethylamino-5,6,7,8-tetrahydronaphthalin-2-yloxy]essigsäureethylester, [(7S)-7(2R)-2-(3-Chlorphenyl)-2-hydroxyethylamino-5,6,7,8-tetrahydronaphthalin-2-yloxy]essigsäureethylester, [2-(3-Chlorphenyl)-2-hydroxyethylamino-5,6,7,8-tetrahydronaphthalin-2-yloxy]essigsäureethylester-hydrochlorid, [(7S)-7(2R)-2-(3-Chlorphenyl)-2-hydroxyethylamino-5,6,7,8-tetrahydronaphthalin-2-yloxy]essigsäureethylester-hydrochlorid oder [(7S)-7(2R)-2-(3-Chlorphenyl)-2-hydroxyethylamino-5,6,7,8-tetrahydronaphthalin-2-yloxy]essigsäureethylester-hydrochlorid in B-Form handelt.

15. Pharmazeutische Zusammensetzung nach einem der Ansprüche 12 bis 14, **dadurch gekennzeichnet, daß** das MARI insbesondere unter Fluoxetin, Fluoxetin-hydrochlorid, Citalopram, Citalopram-hydrobromid, Fluvoxamin, Fluvoxamin-maleat, Paroxetin, Paroxetin-hydrochlorid, Sertralin, Sertralin-hydrochlorid, Milnacipran, Milnacipran-hydrochlorid, Escitalopram (S-Citalopram), Escitalopram-oxalat, Duloxetin, Duloxetin-hydrochlorid, Venlafaxin, Venlafaxin-hydrochlorid, Desvenlafaxin, Radafaxin, Bupropion, Bupropion-hydrochlorid und Mischungen davon ausgewählt ist.

16. Pharmazeutische Zusammensetzung nach Anspruch 15, **dadurch gekennzeichnet, daß** es sich bei dem MARI um Fluoxetin oder Escitalopram handelt.

17. Pharmazeutische Zusammensetzung nach einem der Ansprüche 12 bis 16 zur gleichzeitigen, separaten oder zeitlich gestaffelten Verwendung.

18. Kit, enthaltend einerseits mindestens einen Agonisten von adrenergen β3-Rezeptoren gemäß einem der Ansprüche 1 bis 7 und andererseits mindestens einen MARI gemäß Anspruch 8, wobei die Agonisten von adrenergen β3-Rezeptoren und der MARI in separaten Kompartimenten vorliegen und zur gleichzeitigen, separaten oder zeitlich gestaffelten (sequentiellen) Verabreichung bestimmt sind.

19. Verwendung einer Kombination von mindestens einem Agonisten von adrenergen β3-Rezeptoren, ausgewählt unter Arylethanoldiaminen in Form von Basen oder Säureadditionssalzen, insbesondere 3'-[[2-[[(2R)-2-(3-Chlorphenyl)-2-hydroxyethyl]amino]-ethyl]amino]biphenyl-3-carbonsäure oder dessen Hydrochlorid, und Phenylethanolaminotetralinen der allgemeinen Formel (I) 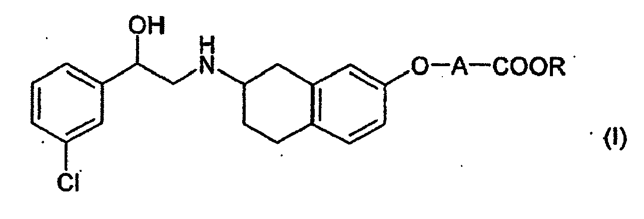 worin:
A für eine C₁₋₄-Alkylengruppe steht und
R für ein Wasserstoffatom oder eine C₁₋₄-Alkylgruppe steht, in Basenform oder Säureadditionssalzform sowie in Hydrat- oder Solvatform, mit mindestens einem MARI zur Herstellung eines Arzneimittels zur Verwendung bei der Behandlung und/oder Prävention von Depression oder Angst.

20. Verwendung nach Anspruch 19, **dadurch gekennzeichnet, daß** die Kombination wie in einem der Ansprüche 2 bis 11 definiert ist.
